# NEUE EUROPÄISCHE PATENTSCHRIFT

(11) **EP 2 247 577 B2**
(45) Veröffentlichungstag und Bekanntmachung des Hinweises auf die Entscheidung über den Einspruch: **27.04.2022**
(45) Hinweis auf die Patenterteilung: 23.12.2015
(21) Anmeldenummer: 09715350.6
(22) Anmeldetag: 12.02.2009
(51) Int. Cl.: C07D 231/12, C07C 251/80

(54) **VERFAHREN ZUR REGIOSELEKTIVEN SYNTHESE VON 1-ALKYL-3-HALOALKYL-PYRAZOL-4-CARBONSÄUR E-DERIVATEN**
METHOD FOR THE REGIOSELECTIVE SYNTHESIS OF 1-ALKYL-3-HALOALKYL-PYRAZOLE-4-CARBOXYLIC ACID DERIVATIVES
PROCÉDÉ DE SYNTHÈSE RÉGIOSÉLECTIVE DE DÉRIVÉS D'ACIDE 1-ALKYL-3-HALOALKYL-PYRAZOL-4-CARBOXYLIQUE

(30) Priorität: 25.02.2008 EP 08151891
(43) Veröffentlichungstag der Anmeldung: 10.11.2010
(73) Patentinhaber: Bayer Intellectual Property GmbH, 40789 Monheim am Rhein (DE)
(72) Erfinder: PAZENOK, Sergii, 42699 Solingen (DE); LUI, Norbert, 51519 Odenthal (DE); HEINRICH, Jens-Dietmar, 51399 Burscheid (DE); WOLLNER, Thomas, 50825 Köln (DE)
(74) Vertreter: BIP Patents
(86) Internationale Anmeldenummer: PCT/EP2009/000958
(87) Internationale Veröffentlichungsnummer: WO 2009/106230

(56) Entgegenhaltungen:
- EP-A1- 1 854 788
- WO-A1-2005/042468
- R.J. ALTENBACH ET AL: JOURNAL OF MEDICINAL CHEMISTRY, AMERICAN CHEMICAL SOCIETY, WASHINGTON, US, Bd. 50, Nr. 22, 1. November 2007 (2007-11-01), Seiten 5439-5448, XP002488452, ISSN: 0022-2623, DOI: DOI:10.1021/JM0705051 [gefunden am 2007-10-06]
- E. OKADA ET AL: HETEROCYCLES, ELSEVIER SCIENCE PUBLISHERS B.V. AMSTERDAM, NL, Bd. 34, Nr. 4, 1. Januar 1992 (1992-01-01) , Seiten 791-798, XP009103121, ISSN: 0385-5414
- C. WAKSELMAN ET AL: JOURNAL OF THE CHEMICAL SOCIETY, CHEMICAL COMMUNICATIONS, Nr. 23, 1975, XP008131157, ISSN: 0022-4936

## Beschreibung

Die vorliegende Erfindung betrifft ein Verfahren zur regioselektiven Synthese von 1-Alkyl-3-haloalkyl-pyrazol-4-carbonsäure-Derivaten durch Cyclisierung von 2,3-disubstituierten Acrylsäure-Derivaten, sowie die bei dem Verfahren als Zwischenprodukte entstehenden Hydrazone.

2-Dihalogenacyl-3-dialkylamino-acrylsäureester der Formel II (Y=COOAlk, Z=O) sind wertvolle Zwischenprodukte für die Herstellung von dihalogenmethyl-substituierten Pyrazolylcarbonsäure-Derivaten, welche wiederum Vorstufen von fungiziden Wirkstoffen darstellen (vgl. WO 03/070705).

Pyrazolcarbonsäure-Derivate werden üblicherweise hergestellt, indem man Acrylsäure-Derivate, die zwei Abgangsgruppen (Z und A) aufweisen, mit Hydrazinen umsetzt.

Bei der Umsetzung mit den Monoalkylhydrazinen erhält man vorwiegend 1-Alkylpyrazole. Allerdings erfolgt die Cyclisierung oftmals nicht regioselektiv. Folglich werden, in Abhängigkeit von Substrat und Reaktionsbedingungen, die unerwünschten 5-Alkylpyrazole in Anteilen zwischen 10 und 80 % gebildet (siehe Schema 1).

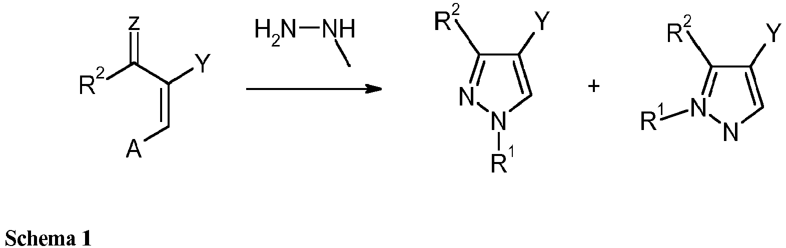

Auch die Synthese von 1-Alkylpyrazolcarbonsäure-Derivaten durch Alkylierung von in 1-Position unsubstituerten Pyrazolderivaten verläuft oftmals unter Bildung beider Regioisomere (siehe Schema 2).

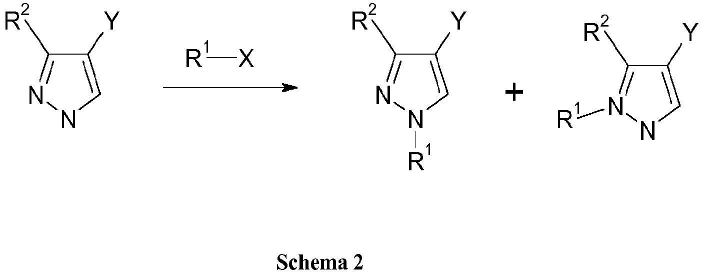

Ein alternativer Weg zur Herstellung von Fluorhaloalkylpyrazolencarbonsäuren ist die Cyclisierung von z. B 4,4-Dichloro-2-[(dimethylamino)methylidene]-3-oxobutanoat mit Alkylhydrazinen, gefolgt von einem Halogenaustausch.

WO 2005/042468 offenbart ein Verfahren zum Herstellen von 2-Dihalogenacyl-3-aminoacrylsäureestern durch Umsetzung von Säurehalogeniden mit Dialkylaminoacrylsäureestern und deren anschließende Cyclisierung mit Alkylhydrazinen

Die bislang unveröffentlichte Europäische Patentanmeldung Nr. 07117232.4 beschreibt ein Verfahren zur Herstellung von HCl-freien 2-Dihalogenacyl-3-amino-acrylsäureestern durch Umsetzung von Säurefluoriden mit Dialkylaminoacrylsäure-Derivaten. Das Verfahren kann in Abwesenheit einer Base durchgeführt werden, wodurch die Abtrennung von Halogenid-Salzen entfällt.

WO 2008/092583 beschreibt ein Verfahren zum Herstellen von 3-Dihalomethyl-pyrazol-4-carbonsäure-Derivaten durch Umsetzung von α,α-Fluoraminen in Gegenwart von Lewissäuren mit Acrylsäure-Derivaten und deren anschließender Umsetzung mit Alkylhydrazinen.

WO 2006/090778 offenbart ein Verfahren zur Herstellung von 1-Methyl-3-difluormethylpyrazolcarbonsäureestern durch Cyclisierung von 2-Alkoxymethylenfluoracylacetat mit Methylhydrazin in Anwesenheit von Wasser und einer Base.

EP 1854788 beschreibt ein regioselektives Verfahren zur Herstellung von 1-Alkyl-3- fluoralkyl-1H-pyrazol-4-carbonsäurealkylestern durch Cyclisierung von 2-Alkoxymethylenfluoracylacetaten mit Alkylhydrazinen in Anwesenheit von Wasser und einer Base.

R.J. ALTENBACH ET AL: JOURNAL OF MEDICINAL CHEMISTRY, AMERICAN CHEMICAL SOCIETY, WASHINGTON, US, Bd. 50, Nr. 22, 1. November 2007 (2007-11-01), Seiten 5439-5448 beschreibt ein Verfahren zur Herstellung von 1-(1,3-Dimethyl-1 H-pyrazol-4-yl)ethanons, worin in einer ersten Stufe 3- Ethoxymethylenpentan-2,4-dion mit 1-Methyl-2-(propan-2-yliden)hydrazon zu 3-((1-Methyl-2-(propan-2-yliden)hydrazinyl) methylen) pentan-2,4-dion umgesetzt wird, das in einer zweiten Stufe cyclisiert wird.

E. OKADA ET AL: HETEROCYCLES, ELSEVIER SCIENCE PUBLISHERS B.V. AMSTERDAM, NL, Bd. 34, Nr. 4, 1. Januar 1992 (1992-01-01), Seiten 791-798 beschreibt ein Verfahren zur Herstellung von 4-Trifluoracetyl-3-trifluormethyl-1-methyl-1H-pyrazol und 4-Trifluoracetyl-3-trifluormethyl-1-phenyl-1H-pyrazol durch Umsetzung von ß,ß-Bis(trifluoracetyl)vinyl-i-butylether mit Benzaldehydmethylhydrazon bzw. Benzaldehydphenylhydrazon zu Benzaldehyd-N-[ß,ß-bis(trifluoracetyl)vinyl]methylhydrazon bzw. Benzaldehyd-N-[ß,ß-bis(trifluoracetyl)vinyl]phenylhydrazon und deren anschließende Cyclisierung.

WO 2005/042468 beschreibt Verfahren zur Herstellung von 2-Dihalogenacyl-3-aminoacrylsäureestern durch Umsetzung von Säurehalogeniden mit Dialkylaminoacrylsäureestern und deren anschließende Cyclisierung mit Alkylhydrazinen zu 1-Alkyl-3-Dihalogenmethyl-1H-pyrazolcarbonsäureestern.

C. WAKSELMAN ET AL: JOURNAL OF THE CHEMICAL SOCIETY, CHEMICAL COMMUNICATIONS, Nr. 23, 1975, offenbart ein Verfahren zur Iminoalkylierung/ Acylierung von elektronenreichen aromatischen Verbindungen mit Hilfe von fluorierten Immonium-Salzen, welche erhältlich sind durch Umsetzung von α,α-Difluoraminen mit BF₃.

Die zuvor beschriebenen Verfahren weisen jedoch alle den Nachteil auf, dass die Cyclisierung, selbst bei niedrigen Temperaturen, nur mit unbefriedigender Regioselektivität erfolgt.

Im Lichte des zuvor beschrieben Standes der Technik, liegt der vorliegenden Erfindung die Aufgabe zugrunde, ein Verfahren zur Verfügung zu stellen, dass die zuvor genannten Nachteile nicht aufweist und somit einen regioselektiven Zugang zu 1-Alkyl-3-haloalkyl-pyrazol-4-carbonsäure-Derivaten in hohen Ausbeuten gewährt.

Die zuvor beschriebene Aufgabe wurde gelöst durch ein Verfahren zum Herstellen von 1-Alkyl-3-haloalkyl-pyrazol-4-carbonsäure-Derivaten der Formel (I) 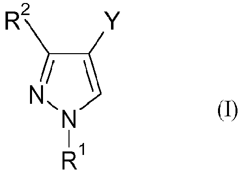 in welcher
- R¹: ausgewählt ist aus Methyl, Ethyl, n-Propyl, iso-Propyl, n-Butyl, sec-Butyl oder tert-Butyl;
- R²: ausgewählt ist aus C₁₋C₄-Alkylgruppen, die mit einem, zwei oder drei Halogenatomen, ausgewählt aus F, Cl und Br oder einer CF₃-Gruppe substituiert sein können;
- Y: ausgewählt ist aus der Gruppe bestehend aus (C=O)OR³, CN und (C=O)NR⁴R⁵, wobei R³, R⁴ und R⁵ unabhängig voneinander ausgewählt sind aus C₁₋₁₂-Alkyl-, C₃₋₋₈-Cycloalkyl-, C₂₋₁₂-Alkenyl-, C₂₋₁₂-Alkinyl-, C₆₋₈-Aryl-, C₇₋₁₉-Arylalkyl- oder C₇₋₁₉-Alkylaryl-Gruppen, die jeweils mit einer oder mehreren Gruppen, die ausgewählt sind aus der Gruppe bestehend aus -R', -X, -OR', -SR', -NR'₂, -SiR'₃, -COOR', -(C=O)R', -CN and -CONR₂', wobei R' Wasserstoff oder eine C₁₋₁₂-Alkyl-Gruppe ist, substituiert sein können und R⁴ und R⁵ zusammen mit dem Stickstoffatom, an das sie gebunden sind, einen 5- bis 6-gliedrigen Ring bilden können, der gegebenenfalls ein oder zwei weitere Hetero-Atome, ausgewählt aus O, S und einer SO₂-Gruppe, enthalten kann und der mit einer oder mehreren Gruppen, die ausgewählt sind aus der Gruppe bestehend aus -R', -X, -OR', -SR', -NR'₂, -SiR'₃, -COOR', -(C=O)R', -CN and -CONR₂', wobei R' Wasserstoff oder eine C₁₋₁₂-Alkyl-Gruppe ist, substituiert sein kann;
umfassend wenigstens eine der Reaktionssequenzen, die sich aus den folgenden Schritten (A) und (D), (B) und (D) oder (C) und (D) zusammensetzen:
(A) Umsetzung eines 2-acylierten oder 2-iminoalkylierten Acrylsäure-Derivats der Formel (II), 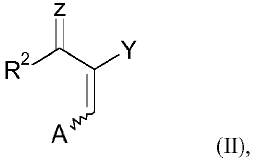 in welcher
   - Z: ausgewählt ist aus O, S und N⁺R¹⁰R¹¹, wobei R¹⁰ und R¹¹ unabhängig voneinander ausgewählt sind aus C₁₋₁₂-Alkyl-, C₃₋₋₈-Cycloalkyl-, C₂₋₁₂-Alkenyl-, C₂₋₁₂-Alkinyl-, C₆₋₈-Aryl-, C₇₋₁₉-Arylalkyl- oder C₇₋₁₉-Alkylaryl-Gruppen, die jeweils mit einer oder mehreren Gruppen, die ausgewählt sind aus der Gruppe bestehend aus -R', -X, -OR', -SR', -NR'₂, -SiR'₃, -COOR', -(C=O)R', -CN and -CONR₂', wobei R' Wasserstoff oder eine C₁₋₁₂-Alkyl-Gruppe ist, substituiert sein können und R¹⁰ und R¹¹ zusammen mit dem Stickstoffatom, an das sie gebunden sind, einen 5- bis 6-gliedrigen Ring bilden können, der gegebenenfalls ein oder zwei weitere Hetero-Atome, ausgewählt aus O, S und einer SO₂-Gruppe, enthalten kann und der mit einer oder mehreren Gruppen, die ausgewählt sind aus der Gruppe bestehend aus -R', -X, -OR', -SR', -NR'₂, -SiR'₃, -COOR', - (C=O)R', -CN and -CONR₂', wobei R' Wasserstoff oder eine C₁₋₁₂-Alkyl-Gruppe ist, substituiert sein kann;
   - A: eine AustrittsgruppeNR¹²R¹³ ist, wobei R¹² und R¹³ unabhängig voneinander ausgewählt sind aus C₁₋₁₂-Alkyl-, C₃₋₋₈-Cycloalkyl-, C₂₋₁₂-Alkenyl-, C₂₋₁₂-Alkinyl-, C₆₋₈-Aryl-, C₇₋₁₉-Arylalkyl- oder C₇₋₁₉-Alkylaryl-Gruppen, die jeweils mit einer oder mehreren Gruppen, die ausgewählt sind aus der Gruppe bestehend aus -R', -X, -OR', -SR', -NR'₂, -SiR'₃, -COOR', -(C=O)R', -CN and -CONR₂', wobei R' Wasserstoff oder eine C₁₋₁₂-Alkyl-Gruppe ist, substituiert sein können und R¹² und R¹³ zusammen mit dem Stickstoffatom, an das sie gebunden sind, einen 5- bis 6-gliedrigen Ring bilden können, der gegebenenfalls ein oder zwei weitere Hetero-Atome, ausgewählt aus O, S und einer SO₂-Gruppe, enthalten kann und der mit einer oder mehreren Gruppen, die ausgewählt sind aus der Gruppe bestehend aus -R', -X, -OR', -SR', -NR'₂, -SiR'₃, -COOR', -(C=O)R', -CN and -CONR₂', wobei R' Wasserstoff oder eine C₁₋₁₂-Alkyl-Gruppe ist, substituiert sein kann;
   mit einem N-Alkyl-Hydrazon der Formel (III) 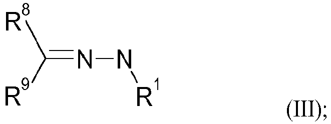 in welcher
   R⁸ ausgewählt ist aus H, Methyl, Ethyl, n-Propyl, iso-Propyl, n-Butyl, sec-Butyl, tert-Butyl, Phenyl, Tolyl, Cyclohexyl;
   R⁹ ausgewählt ist aus H, Methyl, Ethyl, n-Propyl, iso-Propyl, n-Butyl, sec-Butyl, tert-Butyl, Phenyl, Tolyl, Cyclohexyl;
   wobei R⁸ und R⁹ gemeinsam mit dem C-Atom, an das sie geknüpft sind, einen 5- oder 6-gliedrigen Ring bilden können;
(B) Acylierung eines Acrylsäurehydrazons der Formel (VI); 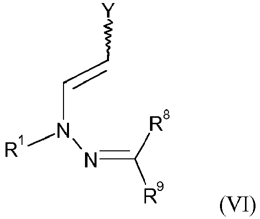 mit einem Acylhalogenid der Formel (X), wobei X ausgewählt ist aus F, Cl, Br oder I, 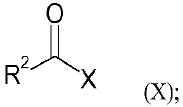
(C) Iminoalkylierung eines Acrylsäurehydrazons der obigen Formel (VI) mit einem α,α-Dihalogenamin der Formel (XI), wobei X ausgewählt ist aus F, Cl, Br oder I, 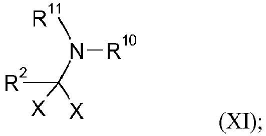
(D) Cyclisierung der in Schritt (A), (B) oder (C) erhaltenen Zwischenprodukte zum 3-Haloalkyl-pyrazol-4-carbonsäure-Derivat der Formel (I).

Überraschenderweise lassen sich die 1-Alkyl-3-haloalkyl-pyrazol-4-carbonsäure-Derivate der Formel (I) unter den erfindungsgemäßen Bedingungen mit guten Ausbeuten, Regioselektivitäten und in hoher Reinheit herstellen, womit das erfindungsgemäße Verfahren die oben genannten Nachteile der im Stand der Technik vorbeschriebenen Herstellungsverfahren überwindet.

### Allgemeine Definitionen

Im Zusammenhang mit der vorliegenden Erfindung umfasst der Begriff Halogene (X), soweit nicht anders definiert, solche Elemente, die ausgewählt sind aus der Gruppe bestehend aus Fluor, Chlor, Brom und Iod, wobei Fluor, Chlor und Brom bevorzugt und Fluor und Chlor besonders bevorzugt verwendet werden.

Gegebenenfalls substituierte Gruppen können einfach oder mehrfach substituiert sein, wobei bei Mehrfachsubstitutionen die Substituenten gleich oder verschieden sein können.

Mit einem oder mehreren Halogenatomen (-X) substituierte Alkyl-Gruppen sind beispielsweise ausgewählt aus Trifluormethyl (CF₃), Difluormethyl (CHF₂), CF₃CH₂, ClCH₂, CF₃CCl₂.

Alkyl-Gruppen sind im Zusammenhang mit der vorliegenden Erfindung, soweit nicht abweichend definiert, lineare oder verzweigte Kohlenwasserstoff-Gruppen, die optional ein, zwei oder mehrere Heteroatome, die ausgewählt sind aus O, N, P und S aufweisen können. Außerdem können die erfindungsgemäßen Alkyl-Gruppen optional durch weitere Gruppen substituiert sein, die ausgewählt sind aus -R', Halogen- (-X), Alkoxy- (-OR'), Thioether- oder Mercapto-(-SR'), Amino-(-NR'₂), Silyl- (-SiR'₃), Carboxyl- (-COOR'), Cyan- (-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Cycloalkyl-Gruppen sind im Zusammenhang mit der vorliegenden Erfindung, soweit nicht abweichend definiert, monocyclische, gesättigte Kohlenwasserstoffgruppen mit 3 bis 8 Kohlenstoffringgliedern, die optional ein, zwei oder mehrere Heteroatome, die ausgewählt sind aus O, N, P und S aufweisen können. Außerdem können die erfindungsgemäßen Cycloalkyl-Gruppen optional durch weitere Gruppen substituiert sein, die ausgewählt sind aus -R', Halogen-(-X), Alkoxy- (-OR'), Thioether- oder Mercapto- (-SR'), Amino- (-NR'₂), Silyl- (-SiR'₃), Carboxyl- (-COOR'), Cyan- (-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Im Einzelnen umfasst diese Definition beispielsweise die Bedeutungen Cyclopropyl, Cyclobutyl, Cyclopentyl und Cyclohexyl.

Die Definition C₁-C₁₂-Alkyl umfasst den größten hierin definierten Bereich für einen Alkyl-Gruppe. Im Einzelnen umfasst diese Definition beispielsweise die Bedeutungen Methyl, Ethyl, n-, iso-Propyl, n-, iso-, sec- und t-Butyl, n-Pentyl, n-Hexyl, 1,3-Dimethylbutyl, 3,3-Dimethylbutyl, n-Heptyl, n-Nonyl, n-Decyl, n-Undecyl, n-Dodecyl.

Alkenyl-Gruppen sind im Zusammenhang mit der vorliegenden Erfindung, soweit nicht abweichend definiert, lineare oder verzweigte Kohlenwasserstoff-Gruppen, die wenigstens eine einfache Unsättigung (Doppelbindung) enthalten und optional eine, zwei oder mehrere einfache oder zweifache Unsättigungen oder ein, zwei oder mehrere Heteroatome, die ausgewählt sind aus O, N, P und S aufweisen können. Außerdem können die erfindungsgemäßen Alkenyl-Gruppen optional durch weitere Gruppen substituiert sein, die ausgewählt sind aus -R', Halogen- (-X), Alkoxy- (-OR'), Thioether- oder Mercapto- (-SR'), Amino- (-NR'₂), Silyl- (-SiR'₃), Carboxyl-(-COOR'), Cyan- (-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₁-C₁₂-Alkenyl umfasst den größten hierin definierten Bereich für einen AlkenylGruppe. Im Einzelnen umfasst diese Definition beispielsweise die Bedeutungen Vinyl; Allyl (2-Propenyl), Isopropenyl (1-Methylethenyl); But-1-enyl (Crotyl), But-2-enyl, But-3-enyl; Hex-1-enyl, Hex-2-enyl, Hex-3-enyl, Hex-4-enyl, Hex-5-enyl; Hept-1-enyl, Hept-2-enyl, Hept-3-enyl, Hept-4-enyl, Hept-5-enyl, Hept-6-enyl; Oct-1-enyl, Oct-2-enyl, Oct-3-enyl, Oct-4-enyl, Oct-5-enyl, Oct-6-enyl, Oct-7-enyl; Non-1-enyl, Non-2-enyl, Non-3-enyl, Non-4-enyl, Non-5-enyl, Non-6-enyl, Non-7-enyl, Non-8-enyl; Dec-1-enyl, Dec-2-enyl, Dec-3-enyl, Dec-4-enyl, Dec-5-enyl, Dec-6-enyl, Dec-7-enyl, Dec-8-enyl, Dec-9-enyl; Undec-1-enyl, Undec-2-enyl, Undec-3-enyl, Undec-4-enyl, Undec-5-enyl, Undec-6-enyl, Undec-7-enyl, Undec-8-enyl, Undec-9-enyl, Undec-10-enyl; Dodec-1-enyl, Dodec-2-enyl, Dodec-3-enyl, Dodec-4-enyl, Dodec-5-enyl, Dodec-6-enyl, Dodec-7-enyl, Dodec-8-enyl, Dodec-9-enyl, Dodec-10-enyl, Dodec-11-enyl; Buta-1,3-dienyl, Penta-1,3-dienyl.

Cycloalkenyl-Gruppen sind im Zusammenhang mit der vorliegenden Erfindung, soweit nicht abweichend definiert, monocyclische, nicht aromatische Kohlenwasserstoffgruppen mit 3 bis 8 Kohlenstoffringgliedern mit mindestens einer Doppelbindung, die optional ein, zwei oder mehrere Heteroatome, die ausgewählt sind aus O, N, P und S aufweisen können. Außerdem können die erfindungsgemäßen Cycloalkenyl-Gruppen optional durch weitere Gruppen substituiert sein, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyan- (-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Im Einzelnen umfasst diese Definition beispielsweise die Bedeutungen Cyclopenten-1-yl, Cyclohexen-1-yl, Cyclohepta-1,3-dien-1-yl.

Alkinyl-Gruppen sind im Zusammenhang mit der vorliegenden Erfindung, soweit nicht abweichend definiert, lineare, verzweigte oder ringförmige Kohlenwasserstoff-Gruppen, die wenigstens eine zweifache Unsättigung (Dreifachbindung) enthalten und optional eine, zwei oder mehrere einfache oder zweifache Unsättigungen oder ein, zwei oder mehrere Heteroatome, die ausgewählt sind aus O, N, P und S aufweisen können. Außerdem können die erfindungsgemäßen Alkinyl-Gruppen optional durch weitere Gruppen substituiert sein, die ausgewählt sind aus -R', Halogen- (-X), Alkoxy- (-OR'), Thioether- oder Mercapto- (-SR'), Amino- (-NR'₂), Silyl-(-SiR'₃), Carboxyl- (-COOR'), Cyan- (-CN), Acyl- (-(C=O)R') und Amid-Gruppen (-CONR₂'), wobei R' Wasserstoff oder eine lineare, verzweigte oder cyclische C₁₋₁₂-Alkyl-Gruppe ist, die ein oder mehrere Heteroatome, ausgewählt aus N, O, P und S, aufweisen kann.

Die Definition C₂-C₁₂-Alkinyl umfasst den größten hierin definierten Bereich für einen Alkinyl-Gruppe. Im Einzelnen umfasst diese Definition beispielsweise die Bedeutungen Ethinyl (Acetylenyl); Prop-1-inyl und Prop-2-inyl.

Aryl-Gruppen sind im Zusammenhang mit der vorliegenden Erfindung, soweit nicht abweichend definiert, aromatische Kohlenwasserstoff-Gruppen, die ein, zwei oder mehrere Heteroatome, die ausgewählt sind aus O, N, P und S aufweisen können und optional durch weitere Gruppen substituiert sein können, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyan- (-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₅₋₁₈-Aryl umfasst den größten hierin definierten Bereich für eine Aryl-Gruppe mit 5 bis 18 Gerüst-Atomen, wobei die C-Atome gegen Heteroatome ausgetauscht sein können. Im Einzelnen umfasst diese Definition beispielsweise die Bedeutungen Cyclopentadienyl, Phenyl, Cycloheptatrienyl, Cyclooctatetraenyl, Naphthyl und Anthracenyl; 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl; 1-Pyrrolyl, 1-Pyrazolyl, 1,2,4-Triazol-1-yl, 1-Imidazolyl, 1,2,3-Triazol-1-yl, 1,3,4-Triazol-1-yl; 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl.

Arylalkyl-Gruppen (Aralkyl-Gruppen) sind im Zusammenhang mit der vorliegenden Erfindung, soweit nicht abweichend definiert, durch Aryl-Gruppen substituierte Alkyl-Gruppen, die eine C₁₋₈-Alkylenkette aufweisen können und im Arylgerüst oder der Alkylenkette durch eines oder mehrere Heteroatome, die ausgewählt sind aus O, N, P und S und optional durch weitere Gruppen substituiert sein können, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyan-(-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₇₋₁₉-Aralkyl-Gruppe umfasst den größten hierin definierten Bereich für einen Arylalkyl-Gruppe mit insgesamt 7 bis 19 Atomen in Gerüst und Alkylenkette. Im Einzelnen umfasst diese Definition beispielsweise die Bedeutungen Benzyl- und Phenylethyl-.

Alkylaryl-Gruppen (Alkaryl-Gruppen) sind im Zusammenhang mit der vorliegenden Erfindung, soweit nicht abweichend definiert, durch Alkyl-Gruppen substituierte Aryl-Gruppen, die eine C₁₋₈-Alkylenkette aufweisen können und im Arylgerüst oder der Alkylenkette durch eines oder mehrere Heteroatome, die ausgewählt sind aus O, N, P und S und optional durch weitere Gruppen substituiert sein können, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyan-(-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₇₋₁₉-Alkylaryl-Gruppe umfasst den größten hierin definierten Bereich für einen Alkylaryl-Gruppe mit insgesamt 7 bis 19 Atomen in Gerüst und Alkylenkette. Im Einzelnen umfasst diese Definition beispielsweise die Bedeutungen Tolyl-, 2,3-, 2,4-, 2,5-, 2,6-, 3,4- oder 3,5-Dimethylphenyl.

Die Alkyl-, Alkenyl-, Alkinyl-, Aryl,- Alkaryl- und Aralkylgruppen können zudem ein oder mehrere Heteroatome aufweisen, die - soweit nicht abweichend definiert - ausgewählt sind aus N, O, P und S. Die Heteroatome ersetzen dabei die bezifferten Kohlenstoffatome.

Die erfindungsgemäßen Verbindungen können gegebenenfalls als Mischungen verschiedener möglicher isomerer Formen, insbesondere von Stereoisomeren, wie z.B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen offenbart und beansprucht.

Über die einzelnen Varianten des erfindungsgemäßen Verfahrens, soll das folgende Schema 3 einen Überblick geben:

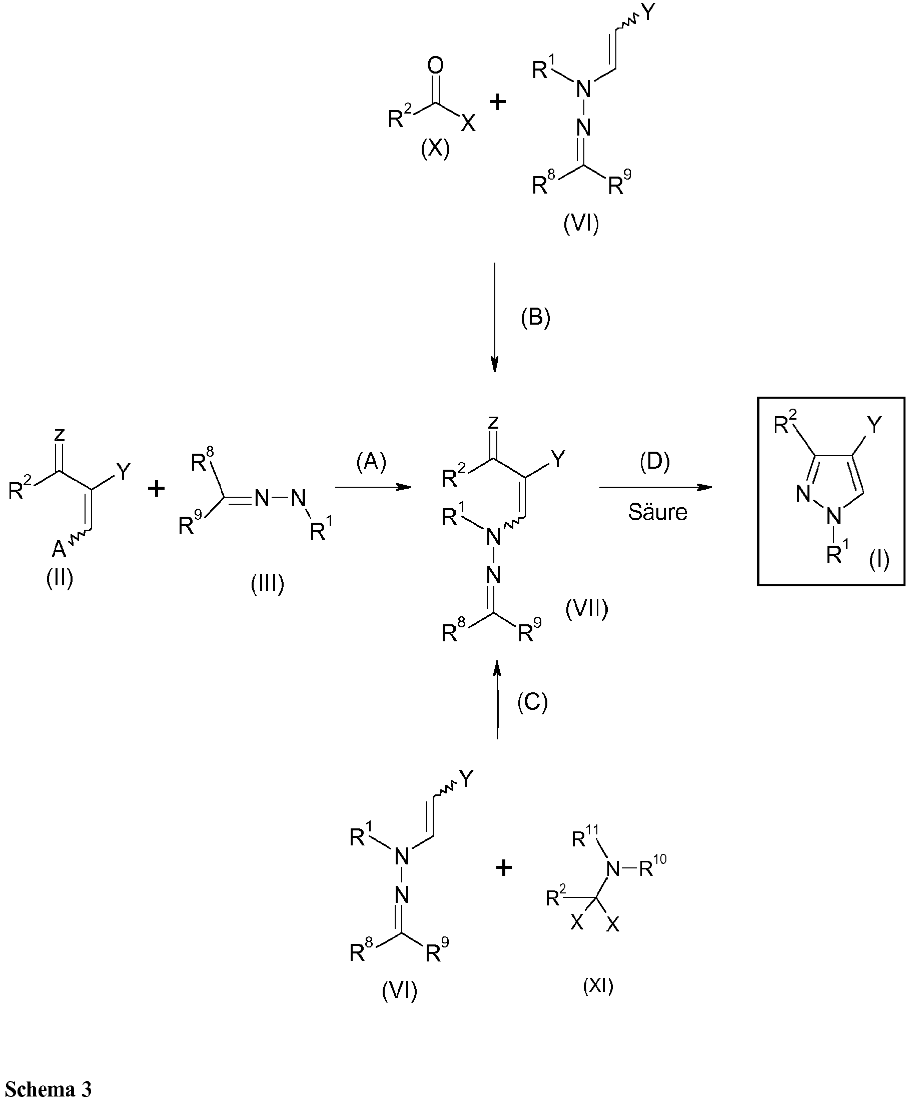

Die gemäß dem erfindungsgemäßen Verfahren erhältlichen 1-Alkyl-3-haloalkyl-pyrazol-4-carbonsäure-Derivaten sind Verbindungen der Formel (I)

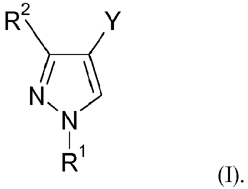

Die Reste in Formel (I) haben erfindungsgemäß die oben genannten Bedeutungen.

In einer *bevorzugten* Ausführungsform der vorliegenden Erfindung haben die Reste in Formel (I) die folgenden Bedeutungen:
- R¹: ist ausgewählt aus Methyl, Ethyl, n-Propyl oder Isopropyl,
- R²: ist ausgewählt aus Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl und 1,1,1-Trifluorprop-2-yl; 1,2,2,2-Tetrafluorethyl,
- Y: ist ausgewählt aus der Gruppe bestehend aus (C=O)OR³, CN und (C=O)NR⁴R^{5,} wobei R³, R⁴ und R⁵ unabhängig voneinander ausgewählt sind aus Methyl, Ethyl, n-Propyl oder Isopropyl.

In einer besonders bevorzugten Ausführungsform der vorliegenden Erfindung haben die Reste in Formel (I) die folgenden Bedeutungen:
- R¹: ist Methyl,
- R²: ist ausgewählt aus Trifluormethyl oder Difluormethyl
- Y: ist ausgewählt aus der Gruppe bestehend aus (C=O)OR³, wobei R³ Methyl oder Ethyl ist.

### Schritt (A)

In einer ersten Ausführungsform des vorliegenden Verfahrens, welche sich durch die Kombination der Reaktionsschritte (A) und (D) ergibt, werden zunächst 2-acylierte oder 2-iminoalkylierte Acrylsäure-Derivate der Formel (II) mit N-Alkylhydrazonen der Formel (III) umgesetzt (Schema 4). Im Anschluss daran werden die in Schritt (A) gebildeten Zwischenprodukte zu den 3-Haloalkyl-pyrazol-4-carbonsäure-Derivaten der Formel (I) cyclisiert (Schritt D).

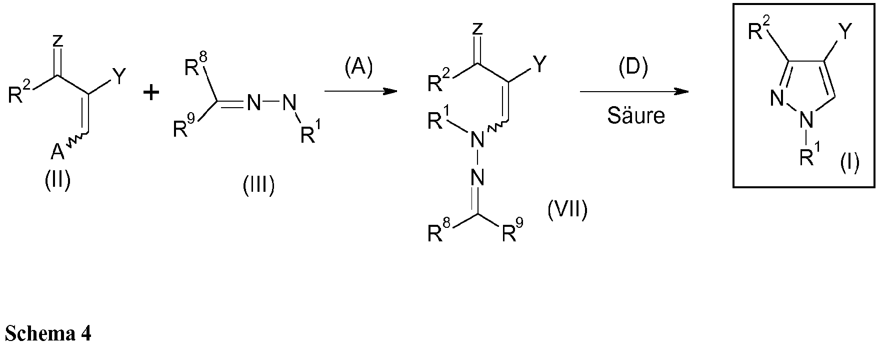

Mögliche Zwischenprodukte, die sich gemäß der vorliegenden Erfindung aus dem Reaktionsschritt (A) ergeben können, sind die Acrylhydrazone gemäß Formel (VII)

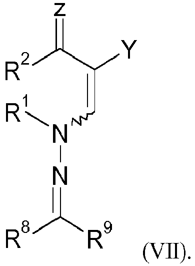

Unter diese Formel fallen insbesondere die 2-Acylhydrazinoprop-2-enoate der Formel (VIIa) und die Hydrazinoaminium Salze der Formel (VIIb)

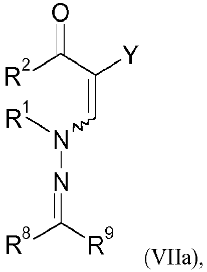

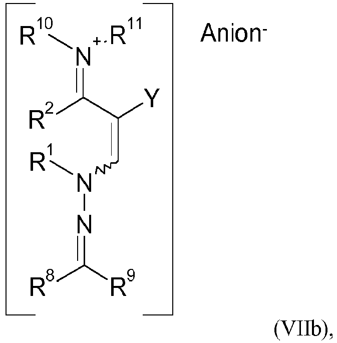

in denen die Reste R¹, R², R⁸ bis R¹¹ und Y die zuvor beschriebenen Bedeutungen haben und die ggf. ein Gegenion (Anion⁻) aufweisen, das ausgewählt ist aus der Gruppe bestehend aus Cl⁻, BF₄⁻, PF₆⁻, SbF₆⁻ und AlCl₄⁻.

Die Durchführung des erfindungsgemäßen Verfahrensschritts (A) erfolgt vorzugsweise innerhalb eines Temperaturbereichs von -20°C bis +150°C, besonders bevorzugt bei Temperaturen von - 10°C bis +70 °C.

Der erfindungsgemäße Verfahrensschritt (A) wird im Allgemeinen unter Normaldruck durchgeführt. Alternativ ist es jedoch auch möglich, im Vakuum zu arbeiten, um die leicht flüchtigen Dialkylamine zu entfernen.

Die Reaktionszeit ist nicht kritisch und kann, in Abhängigkeit von der Ansatzgröße und Temperatur, in einem Bereich zwischen wenigen Minuten und mehreren Stunden gewählt werden.

Bei der Durchführung des erfindungsgemäßen Verfahrensschritts (A) setzt man 1 Mol des Acrylsäure-Derivats der Formel (II) mit 0.5 Mol bis 3 Mol, vorzugsweise 0.5 Mol bis 1.5 Mol, besonders bevorzugt mit der äquimolaren Menge des Hydrazons der Formel (III) um.

Vorzugsweise wird das im Lösungsmittel gelöste Hydrazon der Formel (III) vorgelegt und das Acrylsäure-Derivat der Formel (II) zugegeben. Die umgekehrte Reihenfolge ist jedoch auch möglich.

Die Reaktion kann durch der Zusatz von Katalysatoren wie beispielsweise CH₃COOH, H₂SO₄, KHSO₄, NaH₂PO₄, HCl, CF₃COOH, CH₃COONa beschleunigt werden.

Eine Aufarbeitung und Isolierung der Zwischenprodukte ist im Allgemeinen nicht erforderlich, so dass die aus Schritt (A) erhältliche Reaktionsmischung sofort oder nach erfolgter Zwischenlagerung in den Cyclisierungsschritt (D) eingesetzt werden kann.

Die Acrylsäure-Derivate der Formel (II) sind erhältlich nach den zuvor, im Zusammenhang mit dem Stand der Technik, beschriebenen Verfahren.

Im Zusammenhang mit der vorliegenden Erfindung werden vorzugsweise 2-acylierte oder 2-iminoalkylierte Acrylsäure-Derivate der Formel (II) verwendet, die ausgewählt sind aus der Gruppe bestehend aus, N-1-(Trifluormethyl)-3-(dimethylamino)-2-(ethoxycarbonyl)prop-2-en-1-ylidene]-N-methylmethanaminiumchlorid, 2-(, N-1-(Difluormethyl)-3-(dimethylamino)-2-(ethoxycarbonyl)prop-2-en-1-ylidene]-N-methylmethanaminium tetrafluorborat und N-[3-(Dimethylamino)-2-(ethoxycarbonyl)-1-(1,1,2,2-tetrafluoroethyl)prop-2-en-1-ylidene]-N-methylmethanaminiumchlorid.

Die N-Alkyl-Hydrazone der Formel (III) sind im Zusammenhang mit der vorliegenden Erfindung vorzugsweise ausgewählt aus der Gruppe bestehend aus 1-Methyl-2-(1-methylethylidene)hydrazin, 1-Methyl-2-(1,2,2-trimethylpropylidene)hydrazin, 1-Methyl-2-(1-methylpropylidene)hydrazin, 1-Cyclohexylidene-2-methylhydrazin, 1-Methyl-2-(phenylmethylidene)hydrazin, 1-Methyl-2-(1-phenylethylidene)hydrazin, 1-(Diphenylmethylidene)-2-methylhydrazin, Ethyl-2-[(dimethylamino)methylidene]-4,4,4-trifluoro-3-oxo-butanoat. Die N-Alkyl-Hydrazone der Formel (III) sind in der Literatur vorbeschrieben (Zhurnal Organicheskoi Khimii (1968), 4(6), 986-92.) und durch Umsetzung von kommerziell erhältlichen Hydrazinen der Formel (VIII) mit Carbonylverbindungen der Formel (IX) zugänglich (siehe Schema 5).

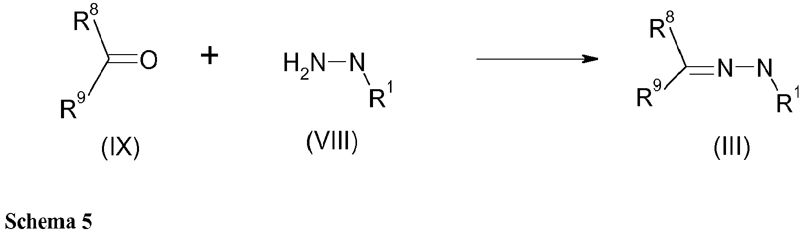

Die Reste in Formeln (III), (VIII) und (IX) haben erfindungsgemäß die folgenden Bedeutungen:
- R¹: ist Methyl, Ethyl, n-Propyl, iso-Propyl, n-Butyl, sec-Butyl, tert-Butyl;
- R⁸: ist ausgewählt aus H, Methyl, Ethyl, n-Propyl, iso-Propyl, n-Butyl, sec-Butyl, tert-Butyl, Phenyl, Tolyl, Cyclohexyl;
- R⁹: ist ausgewählt aus H, Methyl, Ethyl, n-Propyl, iso-Propyl, n-Butyl, sec-Butyl, tert-Butyl, Phenyl, Tolyl, Cyclohexyl.

Alternativ können R⁸ und R⁹ gemeinsam mit dem C-Atom, an das sie geknüpft sind, einen 5- oder 6-gliedrigen Ring bilden.

Die Reste in Formeln (III), (VIII) und (IX) haben erfindungsgemäß die folgenden besonders *bevorzugten* Bedeutungen:
- R¹: ist Methyl;
- R⁸: ist ausgewählt aus H, Methyl, tert-Butyl, Phenyl;
- R⁹: ist ausgewählt aus H, Methyl, tert-Butyl, Phenyl.

Erfindungsgemäß werden bevorzugt Ketone der Formel (IX) verwendet, die ausgewählt sind aus der Gruppe bestehend aus Aceton, Benzophenon, Pinakolon, Cyclohexanon, Benzaldehyd, wobei Aceton und Benzaldehyd besonders bevorzugt verwendet werden.

Ein weiterer Vorteil des erfindungsgemäßen Verfahrens, ist die Tatsache, dass zur Herstellung der N-Methylhydrazone der Formel (III) wässrige Methylhydrazinlösungen verwendet werden können und nicht zwangsläufig das explosive, auch als Raketentreibstoff verwendete, konzentrierte Methylhydrazin eingesetzt werden muss.

In zwei weiteren Ausführungsformen des vorliegenden Verfahrens, welche sich durch die Kombination der Reaktionsschritte (B) und (D) oder (C) und (D) ergibt, werden Acrylsäure-Derivate der Formel (VI) acyliert (Schritt (B)) oder iminoalkyliert (Schritt (C)) und abschließend zu den 3-Haloalkyl-pyrazol-4-carbonsäure-Derivaten der Formel (I) cyclisiert (Schritt D).

### Schritt (B): Acylierung

In einer Ausführungsform des erfindungsgemäßen Verfahrens werden die Acrylsäurehydrazone der Formel (VI) mit Haloalkylcarbonsäurehalogeniden der Formel (X), gemäß dem nachfolgenden Schema 6, zu den 2-Acylacrylsäurehydrazonen der Formel (VIIa) acyliert. In Formel (X) hat R² die obigen Bedeutungen, X ist ein Halogenatom, ausgewählt aus F, Cl oder Br, vorzugsweise aus F und Cl, besonders bevorzugt F.

Im Zusammenhang mit der vorliegenden Erfindung wird vorzugsweise ein Haloalkylcarbonsäurehalogenid der Formel (X) als Acylierungsmittel eingesetzt, welches ausgewählt ist aus der Gruppe bestehend aus Difluoressigsäurechlorid, Difluoressigsäureanhydrid, Difluoressigsäurefluorid, Trifluoressigsäurechlorid und Trifluoressigsäurefluorid.

Die in Schritt (B) verwendeten Acrylsäurehydrazone der Formel (VI) sind vorzugsweise ausgewählt aus der Gruppe bestehend aus Ethyl (2E)-3-[1-methyl-2-(propan-2-ylidene)-hydrazinyl]prop-2-enoate, Ethyl (2Z)-3-[1-methyl-2-(propan-2-ylidene)hydrazinyl]prop-2-enoate, Methyl -3-[1-methyl-2-(propan-2-ylidene)hydrazinyl]prop-2-enoate, Propyl -3-[1-methyl-2-(propan-2-ylidene)hydrazinyl]prop-2-enoate, Ethyl (2E)-3-[1-methyl-2-(3,3-dimethylbutan-2-ylidene)hydrazinyl]prop-2-enoate, Methyl 3-[1-methyl-2-(3,3-dimethylbutan-2-ylidene)hydrazinyl]prop-2-enoate, Methy-3-[1-methyl-2-(phenylmethylidene)hydrazinyl]prop-2-enoate, (2E)-3-[1-methyl-2-(phenylmethylidene)hydrazinyl]prop-2-enenitrile und Methyl -3-[1-ethyl-2-(propan-2-ylidene)hydrazinyl]prop-2-enoate.

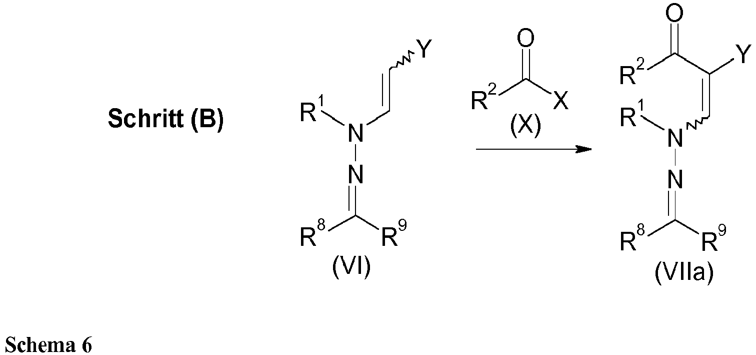

Die Acylierung erfolgt erfindungsgemäß bei Temperaturen von -20°C bis +150°C, bevorzugt bei Temperaturen von -20°C bis +100 °C, besonders bevorzugt bei -10 bis 50°C und unter Normaldruck.

Die Reaktionszeit ist nicht kritisch und kann, in Abhängigkeit von der Ansatzgröße und Temperatur, in einem Bereich zwischen wenigen Minuten und mehreren Stunden gewählt werden.

Erfindungsgemäß setzt man 1 Mol des Acrylsäurehydrazons der Formel (VI) mit 0.5 Mol bis 3 Mol, vorzugsweise 1.2 Mol bis 1.5 Mol, besonders bevorzugt mit äquimolaren Mengen des Haloalkylcarbonsäurehalogenids der Formel (X) um.

Vorzugsweise wird das im Lösungsmittel gelöste Hydrazinoacrylsäure-Derivat der Formel (VI) vorgelegt und das Haloalkylcarbonsäurehalogenid der Formel (X) zugegeben. Die umgekehrte Reihenfolge ist jedoch auch möglich.

Die Acylierung erfolgt vorzugsweise in Gegenwart von einer Base. Bevorzugt sind dazu organische Basen wie Trialkylamine, Alkylpyridine, Phosphazene und 1,8-Diazabicyclo[5.4.0]undecen (DBU); Alkalimetallhydroxide wie z.B. Lithium-, Natrium- oder Kaliumhydroxid, Alkalimetallcarbonate (Na₂CO₃, K₂CO₃) und Alkoholate, wie z.B. NaOMe, NaOEt, NaOt-Bu, KOt-Bu.

Geeignete Lösungsmittel sind beispielsweise aliphatische, alicyclische oder aromatische Kohlenwasserstoffe, wie z.B. Petrolether, n-Hexan, n-Heptan, Cyclohexan, Methylcyclohexan, Benzol, Toluol, Xylol oder Decalin, und halogenierte Kohlenwasserstoffe, wie z.B. Chlorbenzol, Dichlorbenzol, Dichlormethan, Chloroform, Tetrachlormethan, Dichlorethan oder Trichlorethan, Ether, wie Diethylether, Diisopropylether, Methyl-tert-butylether, Methyl-tert-amylether, Dioxan, Tetrahydrofuran, 1,2-Dimethoxyethan, 1,2-Diethoxyethan oder Anisol; Nitrile, wie Acetonitril, Propionitril, n- oder iso-Butyronitril oder Benzonitril; Ketone, wie Aceton, Methylethylketon, Methylisobutylketon oder Cyclohexanon; Amide, wie N,N-Dimethylformamid, N,N-Dimethylacetamid, N-Methylformanilid, N-Methylpyrrolidon oder Hexamethylphosphorsäuretriamid; Sulfoxide, wie Dimethylsulfoxid oder Sulfone, wie Sulfolan. Besonders bevorzugt verwendet man Toluol, Xylol, Chlorbenzol, n-Hexan, Cyclohexan oder Methylcyclohexan, ganz besonders bevorzugt Toluol oder Xylol.

Mögliche Zwischenprodukte, die sich gemäß der vorliegenden Erfindung aus dem Reaktionsschritt (B) ergeben können, sind üblicherweise die 2-Acylhydrazinoacrylsäure-Derivate der Formel (VIIa).

Die gebildeten Zwischenprodukte können ohne vorherige Aufarbeitung in den Cyclisierungsschritt (Schritt D) eingesetzt werden.

Alternativ können die Zwischenprodukte durch geeignete Aufarbeitungsschritte und ggf. weitere Aufreinigung isoliert und zu einem späteren Zeitpunkt in die Cyclisierung (Schritt D) eingesetzt werden.

Die Acrylsäurehydrazone der Formel (VI) sind in der Literatur vorbeschrieben (siehe Chem. Ber. 1975, 108 (7), 2161-2170; Bulletin de la Societe Chimique de France (1976), (1-2, Pt. 2), 260-4; und können gemäß den nachfolgenden Schemata 7 oder 8 hergestellt werden.

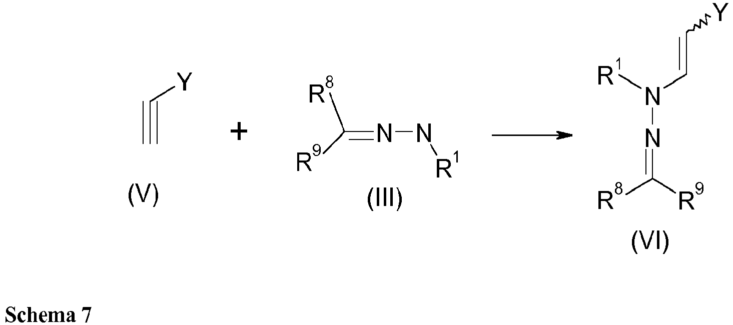

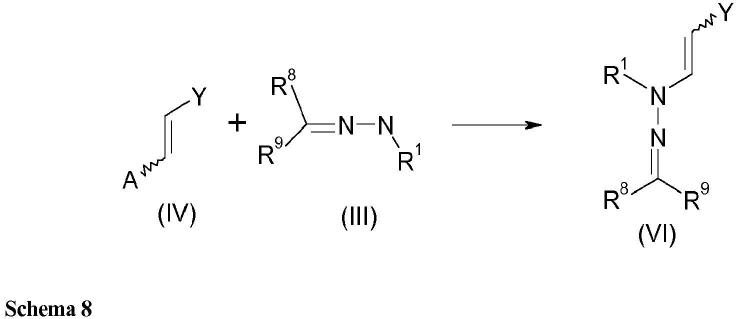

### Schritt (C) Iminoalkylierung

In einer weiteren Ausführungsform des erfindungsgemäßen Verfahrens werden die Acrylsäurehydrazone der Formel (VI) mit wenigstens einem α,α-Dihalogenamin der Formel (XI), gemäß dem nachfolgenden Schema 9, zu den Hydrazinoaminiumsalzen der Formel (VIIb) iminoalkyliert. In Formel (XI) haben X und R² die obigen Bedeutungen.

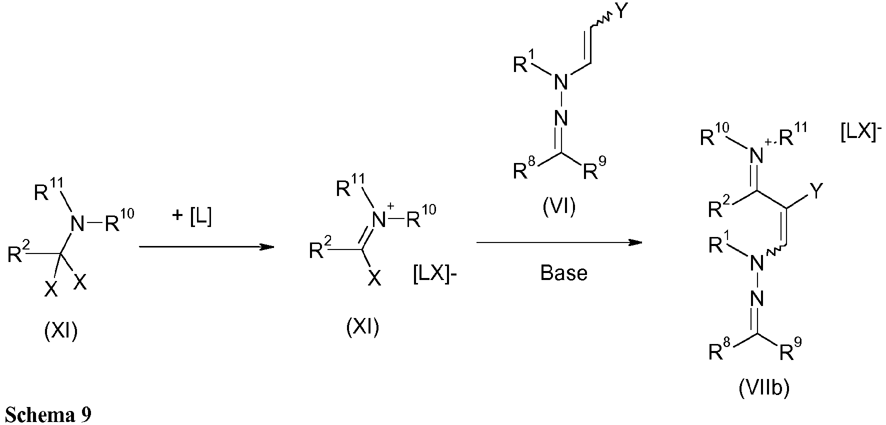

In Schema 9 ist [L] eine Lewissäure.

Im Zusammenhang mit der vorliegenden Erfindung kann 1,1,2,2-Tetrafluorethyl-N,N-dimethylamin, 1,1,2,2-Tetrafluorethyl-N,N-diethylamin, 1,1,2-Trifluor-2-(trifluormethyl)ethyl-N,N-dimethylamin, 1,1,2-Trifluor-2-(trifluormethyl)ethyl-N,N-diethylamin (Ishikawa-Reagenz), 1,1,2-Trifluor-2-chlor-ethyl-N,N-dimethylamin und 1,1,2-Trifluor-2-chlor-ethyl-N,N-diethylamin (Yarovenko-Reagenz), als Iminoalkylierungsmittel der Formel (XI) eingesetzt werden. Dabei sind 1,1,2,2-Tetrafluorethyl-N,N-dimethylamin und 1,1,2,2-Tetrafluorethyl-N,N-diethylamin bevorzugt und 1,1,2,2-Tetrafluorethyl-N,N-dimethylamin besonders bevorzugt.

Die in Schritt (C) verwendeten Acrylsäurehydrazone der Formel (VI) sind vorzugsweise ausgewählt aus der Gruppe bestehend aus Ethyl (2E)-3-[1-methyl-2-(propan-2-ylidene)-hydrazinyl]prop-2-enoate, Ethyl (2Z)-3-[1-methyl-2-(propan-2-ylidene)hydrazinyl]prop-2-enoate, Methyl -3-[1-methyl-2-(propan-2-ylidene)hydrazinyl]prop-2-enoate, Propyl -3-[1-methyl-2-(propan-2-ylidene)hydrazinyl]prop-2-enoate, Ethyl (2E)-3-[1-methyl-2-(3,3-dimethylbutan-2-ylidene)hydrazinyl]prop-2-enoate, Methyl-3-[1-methyl-2-(3,3-dimethylbutan-2-ylidene)hydrazinyl]prop-2-enoate, Methyl-3-[1-methyl-2-(phenylmethylidene)hydrazinyl]prop-2-enoate, (2E)-3-[1-methyl-2-(phenylmethylidene)hydrazinyl]prop-2-enenitrile und Methyl -3-[1-ethyl-2-(propan-2-ylidene)hydrazinyl]prop-2-enoate.

In einer bevorzugten Ausführungsform des erfindungsgemäßen Verfahrens wird das α,α-Dihalogenamin zuerst mit Lewis Säure [L], wie z.B. BF₃, AlCl₃, SbCl₅, SbF₅, ZnCl₂, PF₅ umgesetzt und dann das Gemisch aus Hydrazinoacrylsäureester der Formel (VI) und einer Base, in Substanz oder gelöst in einem geeigneten Lösungsmittel, zugegeben.

Bevorzugt sind organische Basen wie Trialkylamine, Alkylpyridine, Phosphazene und 1,8-Diazabicyclo[5.4.0]undecen (DBU), Alkalimetallhydroxide wie z.B. Lithium-, Natrium- oder Kaliumhydroxid, Alkalimetallcarbonate (Na₂CO₃, K₂CO₃) und Alkoholate, wie z.B. NaOMe, NaOEt, NaOt-Bu, KOt-Bu.

Die Umsetzung der α,α-Dihalogenamine der Formel (XI) mit den Acrylsäurehydrazonen der Formel (VI) erfolgt üblicherweise bei Temperaturen von -50 bis 70°C, vorzugsweise von -20 bis 60°C, besonders bevorzugt von -10 bis 50°C.

Die Reaktion kann unter vermindertem Druck, unter Normal- oder unter Hochdruck, vorzugsweise unter Normaldruck erfolgen.

Die Reaktion kann in Substanz oder in einem Lösungsmittel durchgeführt werden. Vorzugsweise wird die Reaktion in einem Lösungsmittel durchgeführt. Geeignete Lösungsmittel sind beispielsweise ausgewählt aus der Gruppe bestehend aus aliphatischen und aromatischen Kohlenwasserstoffen, wie z.B. n-Hexan, Benzol oder Toluol, die durch Fluor- und Chloratome substituiert sein können, wie Methylenchlorid, Dichlorethan, Fluorbenzol, Chlorbenzol oder Dichlorbenzol; Ethern, wie z.B. Diethylether, Diphenylether, Methyl-tert-butylether, Isopropylethylether, Dioxan, Diglym, Dimethylglycol, Dimethoxyethane (DME) oder THF; Nitrilen wie Methylnitril, Butylnitril oder Phenylnitril; Amide wie Dimethylformamid (DMF) oder N-Methylpyrrolidon (NMP) oder Mischungen solcher Lösungsmittel geeignet, wobei Acetonitril, Dichlormethan, THF, DME und Ethylacetate besonders bevorzugt sind.

Die Base und das Acrylsäurehydrazon der Formel (VI) werden vorzugsweise in äquimolaren Mengen bezogen auf α,α-Dihalogenamine der Formel (XI) eingesetzt. Die Base kann auch im Überschuss eingesetzt werden. Das Verhältnis von Base : Hydrazinoacrylsäure-Derivat liegt erfindungsgemäß zwischen 1,5:1 und 0,9:1, vorzugsweise zwischen 1,4:1 und 0,95:1, besonders bevorzugt zwischen 1,3:1 und 1,05:1.

Aufgrund der Hydrolyseempfindlichkeit der α,α-Dihalogenamine, ist die Umsetzung in wasserfreien Apparaturen unter Inertgasatmosphäre durchzuführen.

Mögliche Zwischenprodukte, die sich gemäß der vorliegenden Erfindung aus dem Reaktionsschritt (C) ergeben können, sind üblicherweise die Hydrazinoaminiumsalze der Formel (VIIb).

Die gebildeten Zwischenprodukte können ohne vorherige Aufarbeitung in den Cyclisierungsschritt (Schritt D) eingesetzt werden.

Alternativ können die Zwischenprodukte durch geeignete Aufarbeitungsschritte und ggf. weitere Aufreinigung isoliert und zu einem späteren Zeitpunkt in die Cyclisierung (Schritt D) eingesetzt werden.

### Schritt(D): Cyclisierung

Die Cyclisierung der aus den Schritten (A), (B) oder (C) erhältlichen Zwischenprodukte erfolgt erfindungsgemäß bei Temperaturen von -20°C bis +150°C, bevorzugt bei Temperaturen von - 10°C bis +100 °C, besonders bevorzugt bei -10 bis 50°C und unter Normaldruck.

Die Reaktionszeit ist nicht kritisch und kann in Abhängigkeit von der Ansatzgröße in einem größeren Bereich gewählt werden.

Erfindungsgemäß wird der Ringschluss zum Pyrazol in Anwesenheit einer Säure durchgeführt. Dabei wird die Carbonylverbindung R⁸R⁹C=O abgespalten. Die Carbonylverbindungen können abgetrennt und wieder für die Herstellung von Hydrazonen der Formel (III) verwendet werden.

Üblicherweise erfolg der Schritt (D) nach den Schritten (A), (B) oder (C) ohne Lösemittelwechsel.

Idealerweise werden alle Reaktionsschritte des erfindungsgemäßen Verfahrens im selben Lösungsmittel durchgeführt.

Geeignete Lösungsmittel sind beispielsweise aliphatische, alicyclische oder aromatische Kohlenwasserstoffe, wie z.B. Petrolether, n-Hexan, n-Heptan, Cyclohexan, Methylcyclohexan, Benzol, Toluol, Xylol oder Decalin, und halogenierte Kohlenwasserstoffe, wie z.B. Chlorbenzol, Dichlorbenzol, Dichlormethan, Chloroform, Tetrachlormethan, Dichlorethan oder Trichlorethan, Ether, wie Diethylether, Diisopropylether, Methyl-tert-butylether, Methyl-tert-amylether, Dioxan, Tetrahydrofuran, 1,2-Dimethoxyethan, 1,2-Diethoxyethan oder Anisol; Nitrile, wie Acetonitril, Propionitril, n- oder iso-Butyronitril oder Benzonitril; Ketone, wie Aceton, Methylethylketon, Methylisobutylketon oder Cyclohexanon; Amide, wie N,N-Dimethylformamid, N,N-Dimethylacetamid, N-Methylformanilid, N-Methylpyrrolidon oder Hexamethylphosphorsäuretriamid; Sulfoxide, wie Dimethylsulfoxid oder Sulfone, wie Sulfolan; Alkohole, wie Methanol, Ethanol, n-oder iso-Propanol, Butanol. Besonders bevorzugt verwendet man Toluol, Xylol, Chlorbenzol, n-Hexan, Cyclohexan oder Methylcyclohexan, Ethanol, ganz besonders bevorzugt Ethanol, Toluol, Xylol und Wasser.

Geeignete Säuren sind ausgewählt aus der Gruppe bestehend aus HCl, H₂SO₄, CF₃COOH, CF₃SO₃H, CH₃COOH, besonders bevorzugt sind HCl und H₂SO₄.

Nach beendeter Reaktion wird beispielsweise die Lösemitteln entfernt und das Produkt durch Filtration isoliert oder zunächst mit Wasser extrahiert, die organische Phase abgetrennt und das Lösungsmittel durch Destillation entfernt.

Das erfindungsgemäße Verfahren soll in den nachstehenden Beispielen weiter beschrieben werden. Die Beispiele sind jedoch nicht in einschränkender Weise zu interpretieren.

### Herstellungsbeispiele

### Beispiel 1: Ethyl-4,4-difluor-2-{[1-methyl-2-(phenylmethyliden)hydrazino]methyliden}-3-oxobutanoat

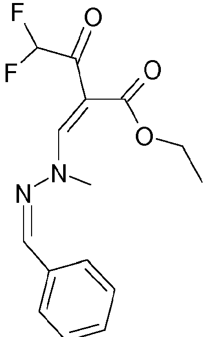

6,2 g 2-Methyl-1-phenylhydrazon und 9,3 g 2-(Difluoracetyl)-3-(dimethylamino)-acrylsäureethyl-ester werden in 100 ml Toluol vorgelegt und mit 5,7 g Kaliumhydrogensulfat versetzt. Anschließend wird die Reaktionsmischung auf 35 - 40 °C erhitzt und 8 h bei gleicher Temperatur gerührt. Nach Abtrennung der Salze wird das Lösungsmittel im Vakuum entfernt. Man erhält Ethyl-4,4-difluor-2-{[1-methyl-2-(phenylmethyliden) hydrazino]methyliden}-3-oxobutanoat als Feststoff (91 % Ausbeute). Das Isomerenverhältnis ist beträgt gemäß NMR 53 / 39 %.

**¹H-NMR** (278 K, CDCN₃): δ= 1,08 (t, 3H); 1,23 (t, 3H); 3,44 (s, 3H); 3,52 (s, 3H); 4,14 (m, 2H); 6,0 (t, 1H); 6,37 (t, 1H); 7,4 - 7,98 (m, 7H) ppm.

## Patentansprüche

1. Verfahren zum Herstellen von 1-Alkyl-3-haloalkyl-pyrazol-4-carbonsäure-Derivaten der Formel (I) 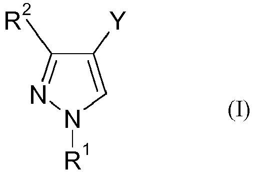 in welcher
R¹ ausgewählt ist aus Methyl, Ethyl, n-Propyl, iso-Propyl, n-Butyl, sec-Butyl oder tert-Butyl;
R² ausgewählt ist aus C₁-C₄-Alkylgruppen, die mit einem, zwei oder drei Halogenatomen, ausgewählt aus F, Cl und Br oder einer CF₃-Gruppe substituiert sein können;
Y ausgewählt ist aus der Gruppe bestehend aus (C=O)OR³, CN und (C=O)NR⁴R⁵, wobei R³, R⁴ und R⁵ unabhängig voneinander ausgewählt sind aus C₁₋₁₂-Alkyl-, C₃₋₋₈-Cycloalkyl-, C₂₋₁₂-Alkenyl-, C₂₋₁₂-Alkinyl-, C₆₋₈-Aryl-, C₇₋₁₉-Arylalkyl- oder C₇₋₁₉-Alkylaryl-Gruppen, die jeweils mit einer oder mehreren Gruppen, die ausgewählt sind aus der Gruppe bestehend aus -R', -X, -OR', -SR', -NR'₂, -SiR'₃, -COOR', -(C=O)R', -CN and -CONR₂', wobei R' Wasserstoff oder eine C₁₋₁₂-Alkyl-Gruppe ist, substituiert sein können und R⁴ und R⁵ zusammen mit dem Stickstoffatom, an das sie gebunden sind, einen 5- bis 6-gliedrigen Ring bilden können, der gegebenenfalls ein oder zwei weitere Hetero-Atome, ausgewählt aus O, S und einer SO₂-Gruppe, enthalten kann und der mit einer oder mehreren Gruppen, die ausgewählt sind aus der Gruppe bestehend aus -R', -X, -OR', -SR', -NR'₂, -SiR'₃, -COOR', -(C=O)R', -CN and -CONR₂', wobei R' Wasserstoff oder eine C₁₋₁₂-Alkyl-Gruppe ist, substituiert sein kann; umfassend wenigstens eine der Reaktionssequenzen, die sich aus den folgenden Schritten (A) und (D), (B) und (D) oder (C) und (D) zusammensetzen:
(A) Umsetzung eines 2-acylierten oder 2-iminoalkylierten Acrylsäure-Derivats der Formel (II), 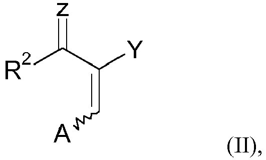 in welcher
Z ausgewählt ist aus O, S und N⁺R¹⁰R¹¹, wobei R¹⁰ und R¹¹ unabhängig voneinander ausgewählt sind aus C₁₋₁₂-Alkyl-, C₃₋₋₈-Cycloalkyl-, C₂₋₁₂-Alkenyl-, C₂₋₁₂-Alkinyl-, C₆₋₈-Aryl-, C₇₋₁₉-Arylalkyl- oder C₇₋₁₉-Alkylaryl-Gruppen, die jeweils mit einer oder mehreren Gruppen, die ausgewählt sind aus der Gruppe bestehend aus -R', -X, -OR', -SR', -NR'₂, -SiR'₃, -COOR', -(C=O)R', -CN and -CONR₂', wobei R' Wasserstoff oder eine C₁₋₁₂-Alkyl-Gruppe ist, substituiert sein können und R¹⁰ und R¹¹ zusammen mit dem Stickstoffatom, an das sie gebunden sind, einen 5-bis 6-gliedrigen Ring bilden können, der gegebenenfalls ein oder zwei weitere Hetero-Atome, ausgewählt aus O, S und einer SO₂-Gruppe, enthalten kann und der mit einer oder mehreren Gruppen, die ausgewählt sind aus der Gruppe bestehend aus -R', -X, -OR', -SR', -NR'₂, -SiR'₃, -COOR', -(C=O)R', -CN and -CONR₂', wobei R' Wasserstoff oder eine C₁₋₁₂-Alkyl-Gruppe ist, substituiert sein kann;
A eine Austrittsgruppe NR¹²R¹³ ist, wobei R¹² und R¹³ unabhängig voneinander ausgewählt sind aus C₁₋₁₂-Alkyl-, C₃₋₋₈-Cycloalkyl-, C₂₋₁₂-Alkenyl-, C₂₋₁₂-Alkinyl-, C₆₋₈-Aryl-, C₇₋₁₉-Arylalkyl- oder C₇₋₁₉-Alkylaryl-Gruppen, die jeweils mit einer oder mehreren Gruppen, die ausgewählt sind aus der Gruppe bestehend aus -R', -X, -OR', -SR', -NR'₂, -SiR'₃, -COOR', -(C=O)R', -CN and -CONR₂', wobei R' Wasserstoff oder eine C₁₋₁₂-Alkyl-Gruppe ist, substituiert sein können und R¹² und R¹³ zusammen mit dem Stickstoffatom, an das sie gebunden sind, einen 5-bis 6-gliedrigen Ring bilden können, der gegebenenfalls ein oder zwei weitere Hetero-Atome, ausgewählt aus O, S und einer SO₂-Gruppe, enthalten kann und der mit einer oder mehreren Gruppen, die ausgewählt sind aus der Gruppe bestehend aus -R', -X, -OR', -SR', -NR'₂, -SiR'₃, -COOR', -(C=O)R', -CN and -CONR₂', wobei R' Wasserstoff oder eine C₁₋₁₂-Alkyl-Gruppe ist, substituiert sein kann;
mit einem N-Alkyl-Hydrazon der Formel (III) 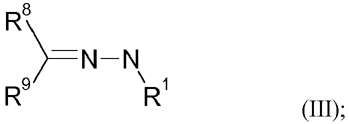 in welcher
R⁸ ausgewählt ist aus H, Methyl, Ethyl, n-Propyl, iso-Propyl, n-Butyl, sec-Butyl, tert-Butyl, Phenyl, Tolyl, Cyclohexyl;
R⁹ ausgewählt ist aus H, Methyl, Ethyl, n-Propyl, iso-Propyl, n-Butyl, sec-Butyl, tert-Butyl, Phenyl, Tolyl, Cyclohexyl;
wobei R⁸ und R⁹ gemeinsam mit dem C-Atom, an das sie geknüpft sind, einen 5- oder 6-gliedrigen Ring bilden können;
(B) Acylierung eines Acrylsäurehydrazons der Formel (VI); 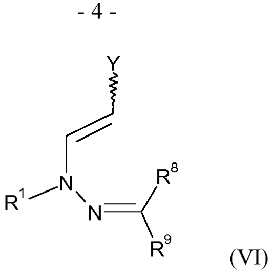 mit einem Acylhalogenid der Formel (X), wobei X ausgewählt ist aus F, Cl, Br oder I; 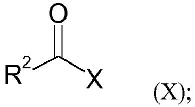
(C) Iminoalkylierung eines Acrylsäurehydrazons der obigen Formel (VI) mit einem α,α-Dihaiogenamin der Formel (XI) , wobei X ausgewählt ist aus F, Cl, Br oder I, 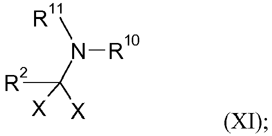
(D) Cyclisierung der in Schritt (A), (B) oder (C) erhaltenen Zwischenprodukte zum 3-Haloalkyl-pyrazol-4-carbonsäure-Derivat der Formel (I).

2. Verfahren gemäß Anspruch 1, **dadurch gekennzeichnet, dass** als Zwischenprodukt der Schritte (A), (B) oder (C) ein 2-acyliertes oder ein 2-iminoalkyliertes Hydrazinoacrylsäure-Derivat der Formel (VII) gebildet wird, 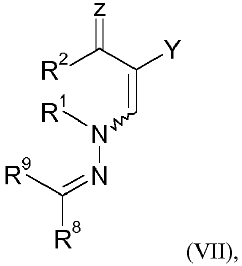 in der die Reste R¹, R², R⁸_{'} R⁹, Y und Z die zuvor beschriebenen Bedeutungen haben.

3. Verfahren gemäß einem der Ansprüche 1 oder 2, **dadurch gekennzeichnet, dass** sich die Reaktionssequenz aus den Schritten (A) und (D) zusammensetzt.

4. Verfahren gemäß einem der Ansprüche 1 oder 2, **dadurch gekennzeichnet, dass** sich die Reaktionssequenz aus den Schritten (B) und (D) zusammensetzt.

5. Verfahren gemäß einem der Ansprüche 1 oder 2, **dadurch gekennzeichnet, dass** sich die Reaktionssequenz aus den Schritten (C) und (D) zusammensetzt.

6. Verfahren gemäß einem der Ansprüche 1 bis 2 oder 4, **dadurch gekennzeichnet, dass** das Acylhalogenid der Formel (X) in Schritt (B) ausgewählt ist aus der Gruppe bestehend aus Difluoressigsäurefluorid oder Difluoressigsäurechlorid.

7. Verfahren gemäß einem der Ansprüche 1 bis 2 oder 5, **dadurch gekennzeichnet, dass** das α,α-Dihalogenamin der Formel (XI) in Schritt (C) 1,1,2,2-Tetrafluorethyl-N,N-dimethylamin ist.

8. Verfahren gemäß einem der Ansprüche 1 bis 3, **dadurch gekennzeichnet, dass** das 2-acylierte oder 2-iminoalkylierte Acrylsäure-Derivat der Formel (II) ausgewählt ist aus der Gruppe bestehend aus N-1-(Trifluormethyl)-3-(dimethylamino)-2-(ethoxycarbonyl)prop-2-en-1-ylidene]-N-methylmethanaminiumchlorid, N-1-(Difluormethyl)-3-(dimethyl-amino)-2-(ethoxycarbonyl)prop-2-en-1-ylidene]-N-methylmethanaminium tetrafluorborat und N-[3-(Dimethylamino)-2-(ethoxycarbonyl)-1-(1,1,2,2-tetrafluoroethyl)prop-2-en-1-ylidene]-N-methylmethanaminiumchlorid.

9. Verfahren gemäß einem der Ansprüche 1 bis 3 oder 8, **dadurch gekennzeichnet, dass** das N-Alkyl-Hydrazon der Formel (III) ausgewählt ist aus der Gruppe bestehend aus 1-Methyl-2-(1-methylethylidene)hydrazin, 1-Methyl-2-(1-phenylethylidene)hydrazin, 1-Methyl-2-(1,2,2-trimethylpropylidene)hydrazin, 1-Methyl-2-(1-methylpropylidene)hydrazin, 1-Cyclohexylidene-2-methylhydrazin, 1-Methyl-2-(phenyl-methylidene)hydrazin, 1-(Diphenylmethylidene)-2-methylhydrazin, Ethyl-2-[(dimethyl-amino)methylidene] -4,4,4-trifluoro-3 -oxobutanoat.

10. Verfahren gemäß einem der Ansprüche 1 bis 2 oder 4 bis 7, **dadurch gekennzeichnet, dass** das Acrylsäurehydrazon der Formel (VI) in den Schritten (B) und (C) ausgewählt ist aus der Gruppe bestehend aus Ethyl (2E)-3-[1-methyl-2-(propan-2-ylidene)hydrazinyl]-prop-2-enoate, Ethyl (2Z)-3-[1-methyl-2-(propan-2-ylidene)hydrazinyl]prop-2-enoate, Methyl -3-[1-methyl-2-(propan-2-ylidene)hydrazinyl]prop-2-enoate, Propyl-3-[1-methyl-2-(propan-2-ylidene)hydrazinyl]prop-2-enoate, Ethyl (2E)-3-[1-methyl-2-(3,3-dimethylbutan-2-ylidene)hydrazinyl]prop-2-enoate, Methyl 3-[1-methyl-2-(3,3-dimethylbutan-2-ylidene)hydrazinyl]prop-2-enoate, Methyl-3-[1-methyl-2-(phenylmethylidene)-hydrazinyl]prop-2-enoate, (2E)-3-[1-methyl-2-(phenylmethylidene)hydrazinyl]prop-2-enenitrile und Methyl -3-[1-ethyl-2-(propan-2-ylidene)hydrazinyl]prop-2-enoate.

11. 2-Acyliertes oder 2-iminoalkyliertes Acrylsäurehydrazon der Formel (VII) 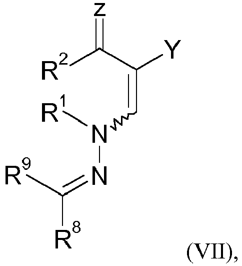 in der die Reste R¹, R², R^{8,} R⁹, Y und Z die zuvor beschriebenen Bedeutungen haben.

12. 2-Acylacrylhydrazon der Formel (VIIa) 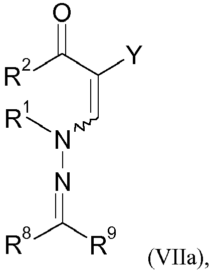 in der die Reste R¹, R², R⁸, R⁹ und Y die zuvor beschriebenen Bedeutungen haben.

13. Salze der Formel (VIIb) 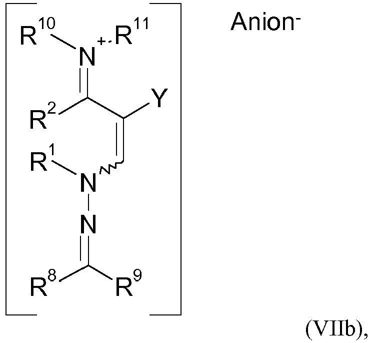 in der die Reste R¹ bis R⁸ und Y die zuvor beschriebenen Bedeutungen haben und das Anion ausgewählt ist aus der Gruppe bestehend aus Cl⁻, BF₄⁻, PF₆⁻, SbF₆⁻ und AlCl₄⁻.

## Claims

1. Process for preparing 1-alkyl-3-haloalkylpyrazole-4-carboxylic acid derivatives of the formula (I) 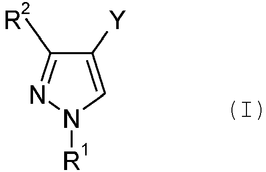 in which
R¹ is selected from methyl, ethyl, n-propyl, isopropyl, n-butyl, sec-butyl and tert-butyl;
R² is selected from C₁-C₄-alkyl groups which may be substituted by one, two or three halogen atoms selected from the group consisting of F, Cl and Br or by a CF₃ group;
Y is selected from the group consisting of (C=O) OR³, CN and (C=O) NR⁴R⁵, where R³, R⁴ and R⁵ independently of one another are selected from the group consisting of C₁₋₁₂-alkyl, C₃₋₈-cycloalkyl, C₂₋₁₂-alkenyl, C₂₋₁₂-alkynyl, C₆₋₈-aryl, C₇₋₁₉-arylalkyl and C₇₋₁₉-alkylaryl groups, each of which may be substituted by one or more groups selected from the group consisting of -R', -X, -OR', -SR', -NR₂, -SiR'₃, -COOR', -(C=O)R', -CN and -CONR₂', where R' is hydrogen or a C₁₋₁₂-alkyl group, and R⁴ and R⁵ together with the nitrogen atom to which they are attached may form a 5- or 6-membered ring which may optionally contain one or two further heteroatoms selected from the group consisting of O, S and an SO₂ group and which may be substituted by one or more groups selected from the group consisting of -R', -X, -OR', -SR', -NR'₂, -SiR₃, -COOR', -(C=O)R', -CN and -CONR₂' , where R' is hydrogen or a C₁₋₁₂-alkyl group; comprising at least one of the reaction sequences consisting of steps (A) and (D), (B) and (D) or (C) and (D) below:
(A) reaction of a 2-acylated or 2-iminoalkylated acrylic acid derivative of the formula (II), 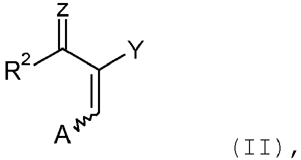 in which
Z is selected from the group consisting of O, S and N⁺R¹⁰R¹¹, where R¹⁰ and R¹¹ independently of one another are selected from the group consisting of C₁₋₁₂-alkyl, C₃₋₈-cycloalkyl, C₂₋₁₂-alkenyl, C₂₋₁₂-alkynyl, C₆₋₈-aryl, C₇₋₁₉-arylalkyl and C₇₋₁₉-alkylaryl groups, each of which may be substituted by one or more groups selected from the group consisting of -R', -X, -OR', -SR', -NR'₂, -SiR'₃, -COOR', - (C=O)R', -CN and -CONR₂' , where R' is hydrogen or a C₁₋₁₂-alkyl group, and R¹⁰ and R¹¹ together with the nitrogen atom to which they are attached may form a 5- or 6-membered ring which may optionally contain one or two further heteroatoms selected from the group consisting of O, S and an SO₂ group and which may be substituted by one or more groups selected from the group consisting of -R', -X, -OR', -SR', -NR'₂, -SiR'₃, -COOR', -(C=O)R', -CN and -CONR₂', where R' is hydrogen or a C₁₋₁₂-alkyl group;
A is a leaving group NR¹²R¹³, where R¹² and R¹³ independently of one another are selected from the group consisting of C₁₋₁₂-alkyl, C₃₋₈-cycloalkyl, C₂₋₁₂-alkenyl, C₂₋₁₂-alkynyl, C₆₋₈-aryl, C₇₋₁₉-arylalkyl and C₇₋₁₉-alkylaryl groups, each of which may be substituted by one or more groups selected from the group consisting of -R', -X, -OR', -SR', -NR₂, -SiR'₃, -COOR', -(C=O)R', -CN and -CONR₂', where R' is hydrogen or a C₁₋₁₂-alkyl group, and R¹² and R¹³ together with the nitrogen atom to which they are attached may form a 5- or 6-membered ring which may optionally contain one or two further heteroatoms selected from the group consisting of O, S and an SO₂ group and which may be substituted by one or more groups selected from the group consisting of -R', -X, -OR', -SR', -NR₂, -SiR'₃, -COOR', -(C=O)R', -CN and -CONR₂', where R' is hydrogen or a C₁₋₁₂-alkyl group; with an N-alkylhydrazone of the formula (III) 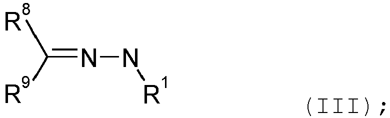 in which
R⁸ is selected from the group consisting of H, methyl, ethyl, n-propyl, isopropyl, n-butyl, sec-butyl, tert-butyl, phenyl, tolyl, cyclohexyl;
R⁹ is selected from the group consisting of H, methyl, ethyl, n-propyl, isopropyl, n-butyl, sec-butyl, tert-butyl, phenyl, tolyl, cyclohexyl,
where R⁸ and R⁹ together with the carbon atom to which they are attached may form a 5- or 6-membered ring;
(B) acylation of an acrylic acid hydrazone of the formula (VI); 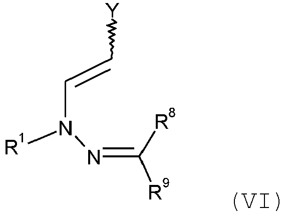 with an acyl halide of the formula (X), where X is selected from the group consisting of F, Cl, Br and I; 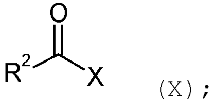
(C) iminoalkylation of an acrylic acid hydrazone of the formula (VI) above with an α,α-dihaloamine of the formula (XI), where X is selected from the group consisting of F, Cl, Br and I, 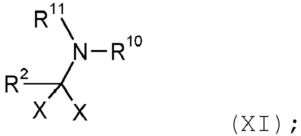
(D) cyclization of the intermediates obtained in step (A), (B) or (C) to give the 3-haloalkylpyrazole-4-carboxylic acid derivative of the formula (I).

2. Process according to Claim 1, **characterized in that**, as intermediate of step (A), (B) or (C), a 2-acylated or a 2-iminoalkylated hydrazinoacrylic acid derivative of the formula (VII) 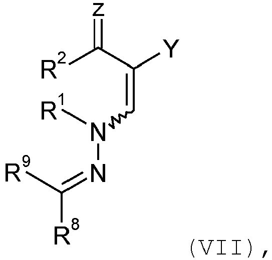 in which the radicals R¹, R², R⁸, R⁹, Y and Z have the meanings described above, is formed.

3. Process according to either of Claims 1 and 2, **characterized in that** the reaction sequence consists of steps (A) and (D).

4. Process according to either of Claims 1 and 2, **characterized in that** the reaction sequence consists of steps (B) and (D).

5. Process according to either of Claims 1 and 2, **characterized in that** the reaction sequence consists of steps (C) and (D).

6. Process according to any of Claims 1 to 2 and 4, **characterized in that** the acyl halide of the formula (X) in step (B) is selected from the group consisting of difluoroacetyl fluoride and difluoroacetyl chloride.

7. Process according to any of Claims 1 to 2 and 5, **characterized in that** the α,α-dihaloamine of the formula (XI) in step (C) is 1,1,2,2-tetrafluoroethyl-N,N-dimethylamine.

8. Process according to any of Claims 1 to 3, **characterized in that** the 2-acylated or 2-iminoalkylated acrylic acid derivative of the formula (II) is selected from the group consisting of N-1-(trifluoromethyl)-3-(dimethylamino)-2-(ethoxycarbonyl)-prop-2-en-1-ylidene]-N-methylmethanaminium chloride, N-1-(difluoromethyl)-3-(dimethylamino)-2-(ethoxycarbonyl)prop-2-en-1-ylidene]-N-methylmethanaminium tetrafluoroborate and N-[3-(dimethylamino)-2-(ethoxycarbonyl)-1-(1,1,2,2-tetrafluoroethyl)prop-2-en-1-ylidene]-N-methylmethanaminium chloride.

9. Process according to any of Claims 1 to 3 and 8, **characterized in that** the N-alkylhydrazone of the formula (III) is selected from the group consisting of 1-methyl-2-(1-methylethylidene)hydrazine, 1-methyl-2-(1-phenylethylidene)hydrazine, 1-methyl-2-(1,2,2-trimethylpropylidene)hydrazine, 1-methyl-2-(1-methylpropylidene)hydrazine, 1-cyclohexylidene-2-methylhydrazine, 1-methyl-2-(phenyl-methylidene)hydrazine, 1-(diphenylmethylidene)-2-methylhydrazine, ethyl 2-[(dimethylamino)methylidene]-4,4,4-trifluoro-3-oxobutanoate.

10. Process according to any of Claims 1 to 2 and 4 to 7, **characterized in that** the acrylic acid hydrazone of the formula (VI) in steps (B) and (C) is selected from the group consisting of ethyl (2E)-3-[1-methyl-2-(propan-2-ylidene)hydrazinyl]prop-2-enoate, ethyl (2Z)-3-[1-methyl-2-(propan-2-ylidene)hydrazinyl]prop-2-enoate, methyl 3-[1-methyl-2-(propan-2-ylidene)hydrazinyl]prop-2-enoate, propyl 3-[1-methyl-2-(propan-2-ylidene)hydrazinyl]prop-2-enoate, ethyl (2E)-3-[1-methyl-2-(3,3-dimethylbutan-2-ylidene)hydrazinyl]prop-2-enoate, methyl 3-[1-methyl-2-(3,3-dimethylbutan-2-ylidene)hydrazinyl]prop-2-enoate, methyl 3-[1-methyl-2-(phenylmethylidene)hydrazinyl]-prop-2-enoate, (2E)-3-[1-methyl-2-(phenylmethylidene)-hydrazinyl]prop-2-enenitrile and methyl 3-[1-ethyl-2-(propan-2-ylidene)hydrazinyl]prop-2-enoate.

11. 2-Acylated or 2-iminoalkylated acrylic acid hydrazone of the formula (VII) 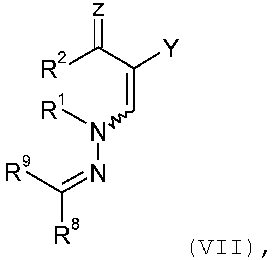 in which the radicals R¹, R², R⁸, R⁹, Y and Z have the meanings described above.

12. 2-Acylacrylic acid hydrazone of the formula (VIIa) 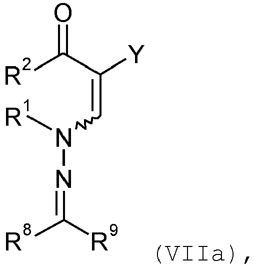 in which the radicals R¹, R², R⁸, R⁹ and Y have the meanings described above.

13. Salts of the formula (VIIb) 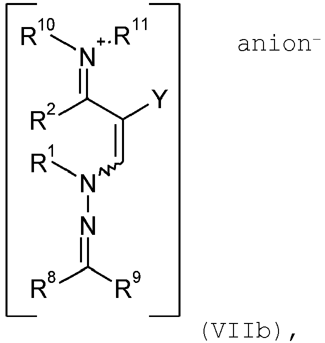 in which the radicals R¹ to R⁸ and Y have the meanings described above and the anion is selected from the group consisting of Cl⁻, BF₄⁻, PF₆⁻, SbF₆⁻ and AlCl₄⁻.

## Revendications

1. Procédé de fabrication de dérivés de l'acide 1-alkyl-3-haloalkyl-pyrazole-4-carboxylique de formule (I) 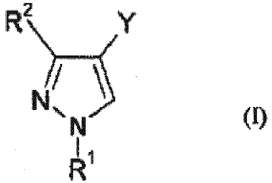 dans laquelle
R¹ est choisi parmi méthyle, éthyle, n-propyle, isopropyle, n-butyle, sec-butyle ou tert-butyle ;
R² est choisi parmi les groupes alkyle en C₁-C₄, qui peuvent être substitués avec un, deux ou trois atomes d'halogène, choisis parmi F, Cl et Br, ou un groupe CF₃ ;
Y est choisi dans le groupe constitué par (C=O)OR³, CN et (C=O)NR⁴R⁵, R³, R⁴ et R⁵ étant choisis indépendamment les uns des autres parmi les groupes alkyle en C₁₋₁₂, cycloalkyle en C₃₋₈, alcényle en C2-12, alcynyle en C2-12, aryle en C₆₋₈, arylalkyle en C7-19 ou alkylaryle en C7-19, qui peuvent chacun être substitués avec un ou plusieurs groupes, qui sont choisis dans le groupe constitué par - R', -X, -OR', -SR', -NR'₂, -SiR'₃, -COOR', -(C=O)R', -CN et -CONR₂', R' représentant l'hydrogène ou un groupe alkyle en C₁₋₁₂, et R⁴ et R⁵ pouvant former ensemble avec l'atome d'azote auquel ils sont reliés un cycle de 5 ou 6 éléments, qui peut éventuellement contenir un ou deux hétéroatomes supplémentaires choisis parmi O, S et un groupe SO₂, et qui peut être substitué avec un ou plusieurs groupes, qui sont choisis dans le groupe constitué par -R', -X, -OR', -SR', -NR'₂, -SiR'₃, -COOR', -(C=O)R', -CN et -CONR₂', R' représentant l'hydrogène ou un groupe alkyle en C₁₋₁₂ ;
comprenant au moins une des séquences réactionnelles composées par les étapes (A) et (D), (B) et (D) ou (C) et (D) suivantes :
(A) la mise en réaction d'un dérivé d'acide acrylique 2-acylé ou 2-iminoalkylé de formule (II) 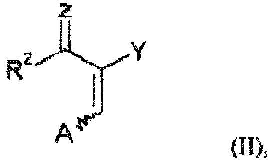 dans laquelle
Z est choisi parmi O, S et N⁺R¹⁰R¹¹, R¹⁰ et R¹¹ étant choisis indépendamment l'un de l'autre parmi des groupes alkyle en C₁₋₁₂,cycloalkyle en C₃₋₈, alcényle en C2-12, alcynyle en C2-12, aryle en C₆₋₈, arylalkyle en C7-19 ou alkylaryle en C₇₋₁₉, qui peuvent chacun être substitués avec un ou plusieurs groupes, qui sont choisis dans le groupe constitué par -R', -X, -OR', -SR', -NR'₂, -SiR'₃, -COOR', -(C=O)R', -CN et -CONR₂', R' représentant l'hydrogène ou un groupe alkyle en C₁₋₁₂, et R¹⁰ et R¹¹ pouvant former ensemble avec l'atome d'azote auquel ils sont reliés un cycle de 5 ou 6 éléments, qui peut éventuellement contenir un ou deux hétéroatomes supplémentaires choisis parmi O, S et un groupe SO₂, et qui peut être substitué avec un ou plusieurs groupes, qui sont choisis dans le groupe constitué par -R', -X, -OR', -SR', -NR'₂, -SiR'₃, -COOR', -(C=O)R', -CN et -CONR₂', R' représentant l'hydrogène ou un groupe alkyle en C₁₋₁₂ ;
A représente un groupe partant NR¹²R¹³, R¹² et R¹³ étant choisis indépendamment l'un de l'autre parmi des groupes alkyle en C₁₋₁₂,cycloalkyle en C₃₋₈, alcényle en C₂₋₁₂, alcynyle en C₂₋₁₂, aryle en C₆₋₈, arylalkyle en C₇₋₁₉ ou alkylaryle en C₇₋₁₉, qui peuvent chacun être substitués avec un ou plusieurs groupes, qui sont choisis dans le groupe constitué par -R', -X, -OR', -SR', -NR'₂, -SiR'₃, -COOR', -(C=O)R', -CN et -CONR₂', R' représentant l'hydrogène ou un groupe alkyle en C₁₋₁₂, et R¹² et R¹³ pouvant former ensemble avec l'atome d'azote auquel ils sont reliés un cycle de 5 ou 6 éléments, qui peut éventuellement contenir un ou deux hétéroatomes supplémentaires choisis parmi O, S et un groupe SO₂, et qui peut être substitué avec un ou plusieurs groupes, qui sont choisis dans le groupe constitué par -R', -X, -OR', -SR', -NR'₂, -SiR'₃, -COOR', -(C=O)R', -CN et -CONR₂', R' représentant l'hydrogène ou un groupe alkyle en C₁₋₁₂ ;
avec une N-alkyl-hydrazone de formule (III) 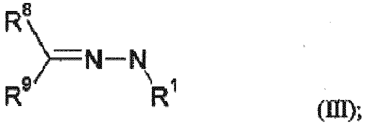 dans laquelle
R⁸ est choisi parmi H, méthyle, éthyle, n-propyle, iso-propyle, n-butyle, sec-butyle, tert-butyle, phényle, tolyle, cyclohexyle ;
R⁹ est choisi parmi H, méthyle, éthyle, n-propyle, iso-propyle, n-butyle, sec-butyle, tert-butyle, phényle, tolyle, cyclohexyle ;
R⁸ et R⁹ pouvant former, ensemble avec l'atome de C auquel ils sont reliés, un cycle de 5 ou 6 éléments ;
(B) l'acylation d'une hydrazone d'acide acrylique de formule (VI) 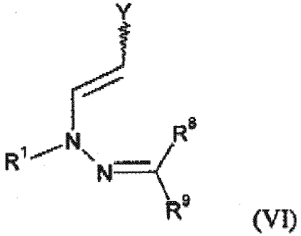 avec un halogénure d'acyle de formule (X), X étant choisi parmi F, Cl, Br ou I ; 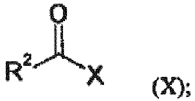
(C) l'iminoalkylation d'une hydrazone d'acide acrylique de la formule (VI) ci-dessus avec une α,α-dihalogénoamine de formule (XI), X étant choisi parmi F, Cl, Br ou I, 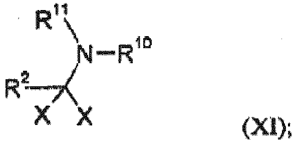
(D) la cyclisation des produits intermédiaires obtenus à l'étape (A), (B) ou (C) pour former le dérivé de l'acide 3-haloalkyl-pyrazole-4-carboxylique de formule (I).

2. Procédé selon la revendication 1, **caractérisé en ce qu'**un dérivé de l'acide hydrazinoacrylique 2-acylé ou 2-iminoalkylé de formule (VII) est formé en tant que produit intermédiaire de l'étape (A), (B) ou (C) 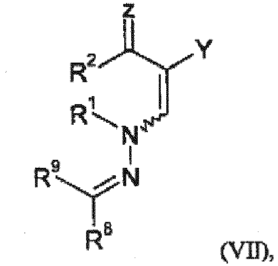 dans laquelle les radicaux R¹, R², R⁸, R⁹, Y et Z ont les significations indiquées précédemment.

3. Procédé selon l'une quelconque des revendications 1 et 2, **caractérisé en ce que** la séquence réactionnelle est composée par les étapes (A) et (D).

4. Procédé selon l'une quelconque des revendications 1 et 2, **caractérisé en ce que** la séquence réactionnelle est composée par les étapes (B) et (D).

5. Procédé selon l'une quelconque des revendications 1 et 2, **caractérisé en ce que** la séquence réactionnelle est composée par les étapes (C) et (D).

6. Procédé selon l'une quelconque des revendications 1 à 2 ou 4, **caractérisé en ce que** l'halogénure d'acyle de formule (X) à l'étape (B) est choisi dans le groupe constitué par le fluorure de l'acide difluoroacétique ou le chlorure de l'acide difluoroacétique.

7. Procédé selon l'une quelconque des revendications 1 à 2 ou 5, **caractérisé en ce que** 1'α,α-dihalogénoamine de formule (XI) à l'étape (C) est la 1,1,2,2-tétrafluoroéthyl-N,N-diméthylamine.

8. Procédé selon l'une quelconque des revendications 1 à 3, **caractérisé en ce que** le dérivé d'acide acrylique 2-acylé ou 2-iminoalkylé de formule (II) est choisi dans le groupe constitué par le chlorure de N-[1-(trifluorométhyl)-3-(diméthylamino)-2-(éthoxycarbonyl)-prop-2-én-1-ylidène]-N-méthylméthanaminium, le tétrafluoroborate de N-[1-(difluorométhyl)-3-(diméthylamino)-2-(éthoxycarbonyl)prop-2-én-1-ylidène]-N-méthylméthanaminium et le chlorure de N-[3-(diméthylamino)-2-(éthoxycarbonyl)-1-(1,1,2,2-tétrafluoroéthyl)prop-2-én-1-ylidène]-N-méthylméthanaminium.

9. Procédé selon l'une quelconque des revendications 1 à 3 ou 8, **caractérisé en ce que** la N-alkyl-hydrazone de formule (III) est choisie dans le groupe constitué par la 1-méthyl-2-(1-méthyléthylidène)hydrazine, la 1-méthyl-2-(1-phényléthylidène)hydrazine, la 1-méthyl-2-(1,2,2-triméthylpropylidène)hydrazine, la 1-méthyl-2-(1-méthylpropylidène)hydrazine, la 1-cyclohexylidène-2-méthylhydrazine, la 1-méthyl-2-(phénylméthylidène)hydrazine, la 1-(diphénylméthylidène)-2-méthylhydrazine, le 2-[(diméthylamino)méthylidène]-4,4,4-trifluoro-3-oxobutanoate d'éthyle.

10. Procédé selon l'une quelconque des revendications 1 à 2 ou 4 à 7, **caractérisé en ce que** l'hydrazone d'acide acrylique de formule (VI) aux étapes (B) et (C) est choisie dans le groupe constitué par le (2E)-3-[1-méthyl-2-(propan-2-ylidène)-hydrazinyl]prop-2-énoate d'éthyle, le (2Z)-3-[1-méthyl-2-(propan-2-ylidène)hydrazinyl]prop-2-énoate d'éthyle, le 3-[1-méthyl-2-(propan-2-ylidène)hydrazinyl]prop-2-énoate de méthyle, le 3-[1-méthyl-2-(propan-2-ylidéne)hydrazinyl]prop-2-énoate de propyle, le (2E)-3-[1-méthyl-2-(3,3-diméthylbutan-2-ylidéne)hydrazinyl]prop-2-énoate d'éthyle, le 3-[1-méthy-2-(3,3-diméthylbutan-2-ylidène)hydrazinyl]prop-2-énoate de méthyle, le 3-[1-méthyl-2-(phénylméthylidène)-hydrazinyl]prop-2-énoate de méthyle, le (2E)-3-[1-méthyl-2-(phénylméthylidène)hydrazinyl] prop-2-ènenitrile et le 3-[1-éthyl-2-(propan-2-ylidène)hydrazinyl]prop-2-énoate de méthyle.

11. Hydrazone d'acide acrylique 2-acylée ou 2-iminoalkylée de formule (VII) 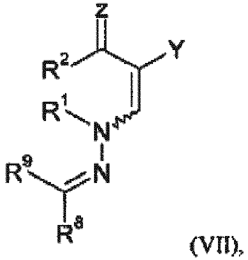 dans laquelle les radicaux R¹, R², R⁸, R⁹, Y et Z ont les significations décrites précédemment.

12. 2-Acylacrylhydrazone de formule (VIIa) 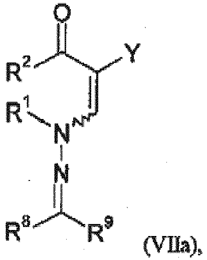 dans laquelle les radicaux R¹, R², R⁸, R⁹ et Y ont les significations décrites précédemment.

13. Sels de formule (VIIb) 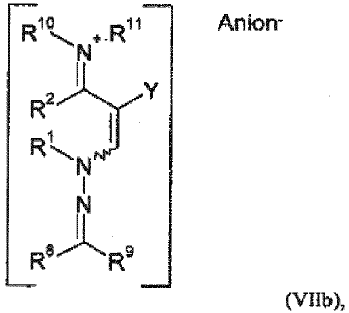 dans laquelle les radicaux R¹ à R⁸ et Y ont les significations décrites précédemment et l'anion est choisi dans le groupe constitué par Cl⁻, BF₄ , PF₆⁻, SbF₆⁻ et AlCl₄⁻.
